# EUROPÄISCHE PATENTSCHRIFT

(11) **EP 4 256 028 B1**
(45) Veröffentlichungstag und Bekanntmachung des Hinweises auf die Patenterteilung: **26.08.2026**
(21) Anmeldenummer: 22738539.0
(22) Anmeldetag: 28.06.2022
(51) Int. Cl.: C12M 1/33, C12M 1/00

(54) **VERFAHREN UND VORRICHTUNG ZUR HYDRODYNAMISCHEN SCHWERSTOFFABTRENNUNG MIT HOHEM WIRKUNGSGRAD**
METHOD AND DEVICE FOR HYDRODYNAMICALLY SEPARATING DENSE MATERIALS WITH A HIGH DEGREE OF EFFICIENCY
PROCÉDÉ ET DISPOSITIF DE SÉPARATION HYDRODYNAMIQUE DE MATÉRIAUX DENSES À HAUT RENDEMENT

(30) Priorität: 11.08.2021 DE 102021004122
(43) Veröffentlichungstag der Anmeldung: 11.10.2023
(73) Patentinhaber: BTA International GmbH, 85276 Pfaffenhofen a. d. Ilm (DE)
(72) Erfinder: KÜBLER, Hans, 80995 München (DE)
(74) Vertreter: K & H Bonapat Patentanwälte Koch · von Behren & Partner mbB
(86) Internationale Anmeldenummer: PCT/DE2022/100470
(87) Internationale Veröffentlichungsnummer: WO 2023/016596

(56) Entgegenhaltungen:
- EP-A1- 0 142 873
- WO-A1-2013/160352
- CN-A- 105 602 844
- DE-A1- 102015 112 254
- DE-A1- 4 120 808
- US-A1- 2015 191 751

## Beschreibung

Die Erfindung betrifft ein Verfahren und eine Vorrichtung zur Abtrennung von Schwerstoffen aus einer Aufschlämmung von Bestandteilen unterschiedlicher Dichte und unterschiedlicher Partikelstruktur in Verbindung mit einer anaeroben Vergärung der vergärbaren Bestandteile der Aufschlämmung. Verfahren und Vorrichtung gewährleisten unabhängig von der Viskosität bzw. des Feststoffgehalts der Aufschlämmung einen hohen Abtrenngrad der Schwerstoffe.

Als ausgehender Stand der Technik sind beispielhaft die DE 10 2015 112254 A1 (Schwerstoffabtrennung), CN 105 602 844 A (Trennverfahren einer Suspension zur MEthanerzeugung), DE 41 20 808 A1 (Aufbereitung von Abfällen) und EP 0 142 873 A1 (Schwerstoffabtrennung eines Fermenters) zu nennen, die jeweils ein Verfahren zur hydrodynamischen Abtrennung von Schwerstoffen einer Aufschlämmung zum Gegenstand haben. Dabei wird sich jeweils eines Hydrozyklons bedient, um eine Abtrennung von Bestandteilen mit unterschiedlicher Dichte oder unterschiedlicher Partikelstruktur auszuschleusen.

Bei einer Aufbereitung von Stoffgemischen mit entsprechender Feuchte, z.B. organischen Abfällen, mechanisch abgetrennten Abfallfraktionen oder Reststoffen aus dem Lebensmittelbereich, entstehen Aufschlämmungen, z.B. Pulpen oder Suspensionen, die noch relevante Mengen an in Wasser sedimentierenden und scharfkantigen Stoffen, z.B. Kies, Split, Steine, Keramik- bzw. Glasbruchstücke oder Metallpartikel, enthalten. Diese Bestandteile verursachen in nachgeschalteten Verfahrensstufen Betriebsprobleme, z.B. Ablagerungen oder Verschleiß. Die Folgen sind beispielsweise Sedimentschichten in Behältern, die ein aufwändiges Entleeren nach wenigen Betriebsjahren erforderlich machen, Verlegen von Rohrleitungen, was einen hohen Reinigungsaufwand verursacht, oder durch die meistens abrasiven Eigenschaften dieser Stoffe ein starker Verschleiß der Maschinentechnik, der einen häufigen Austausch verschlissener Maschinenteile erfordert.

Für eine Vergärung geeignete organische Abfälle können mineralische Schwerstoffe von z.B. 4 Gew.% enthalten (Kübler, H., Hoppenheidt, K., Hirsch, P., Kottmair, A., Nimmrichter, R., Nordsieck, H., M., Mücke, W. , Swerev (2000) Full scale co-digestion of organic waste. Water Science & Technology 41, 195-202). Kommunale Bioabfälle enthalten relevante Mengen an mineralischen Schwerstoffen wie Steine, Glasscherben, Split oder Kies bzw. Sand, die gemäß den Untersuchungen von Kranert et. al. (Kranert, M., Hartmann A., Graul S. (1999) Determination of sand content in digestate. In: W. Bidlingmaier et al. (Hrsg.) Proceedings of the International Conference ORBIT 99 on Biological Treatment of Waste and the Environment, Part I, 313-318) einen Anteil an der Trockenmasse des Abfalls von teilweise über 25 Gew.% ausmachen können. Ein wesentlicher Teil dieser mineralischen Schwerstoffe wird bei einer Aufbereitung der Bioabfälle in den Stoffstrom eingetragen, der dann der biologischen Verwertung zugeführt wird.

Beim Betrieb von Abfallbehandlungsanlagen, in denen die abgesiebte Fraktion kleiner 80 mm einer Aufbereitung zugeführt wird, wurde in dieser Fraktion ein Anteil von Glaspartikeln und mineralischen Bestandteilen von 12 bis 14 Gew.% an der Feuchtmasse dieser Fraktion ermittelt (Rita, J., Braga, J., Mannall, C., Goldsmith, S., Kübler, H., Rahn, T., Schulte, S. (2015) Compost-like material or thermal valorisation - impact on MBT Plant economics and environmental aspects - case studies in Portugal and UK. In: M. Kühle-Weidemeier und M. Balhar (Hrsg.) Energie und Rohstoffe aus Rest- und Bioabfällen, Cuvillier Verlag Göttingen, 395-406).

Um eine störungsarme Verwertung der Suspensionen aus einer Nassaufbereitung sicherzustellen, werden häufig die leicht sedimentierenden Anteile aus der Suspension abgetrennt. Hierzu werden Schwerstoffabscheider eingesetzt. Diese beruhen entweder auf einer Sedimentation im Schwerkraftfeld der Erde oder auf hydrodynamisch erzeugter höherer Beschleunigung, um Verweilzeiten reduzieren zu können. Zentraler Bestandteil der hydrodynamischen Schwerstoffabscheider ist häufig ein Hydrozyklon (Wirbelabscheider).

Ein Wirbelabscheider bestehend aus zwei konzentrischen Rohren wird in GB 739705 beschrieben. Dieser hat keine konischen Abschnitte und die Rohre sind entlang ihrer gemeinsamen Längsachse einstellbar.

DE 6949940 beschreibt einen Wirbelabscheider zum Reinigen von Suspensionen mit tangentialem Einlauf, zum Wirbelrohr koaxialem Auslauf für die gereinigte Suspension und einem kegelig verjüngenden Auslass für die abgeschiedenen Partikel.

DE 3427395 beschreibt ein Verfahren zur Wirbelabscheidung von Verunreinigungen aus Problemschlämmen mit einem Flüssigkeitsbett. Dieses Flüssigkeitsbett wird durch Eintrag von Waschwasser von unten fluidisiert und in der Wirbelabscheidung ausgewaschen. Gröbere Bestandteile werden abgeführt und feinere in die Wirbelabscheidung zurückgeführt. Die Vorrichtung zur Durchführung des Verfahrens besteht aus einem Hydrozyklon mit konischem Unterteil, an das der Waschbehälter angeschlossen ist.

Ein Flachbodenhydrozyklon wird in GB 2076315 beschrieben. Dieser hat einen koaxialen Feststoffaustrag in eine unterhalb angeordnete Speicherkammer für die abgetrennten Feststoffe.

DE 19505073 beschreibt einen Flachbodenhydrozyklon zur Abtrennung von Schwerstoffen aus einer Aufschlämmung, die aus Abfallstoffen erzeugt wurde. Durch die Kombination von Flachbodenzyklon und Klassierrohr wird die Selektivität des Schwerstoffabscheiders erhöht. Die abgetrennten Schwerstoffe werden im Unterlauf des Klassierrohres mittels eines Schleusensystems mit Speicherkammer diskontinuierlich ausgetragen. Eine vergleichbare Vorrichtung wird auch in DE 29502488 beschrieben.

Ein Verfahren und eine Vorrichtung zur Schwerstoffabtrennung in Verbindung mit einem Vorlagebehälter beschreibt EP 1497010. Der Vorlagebehälter wird durch eine Trennwand in zwei Zonen unterteilt. Der einen Zone wird ungereinigte Suspension zugeführt und die mit dem Schwerstoffabscheider gereinigte Suspension wird der anderen Zone zugeführt. Der Schwerstoffabscheider wird aus der Zone mit der ungereinigten Suspension beschickt. Die Zone für die gereinigte Suspension hat einen Abzug quer zur Hauptstromrichtung. Die Trennwand ist so positioniert, dass im Betrieb immer gereinigtes Material in die Zone mit ungereinigtem Material fließt und nicht umgekehrt.

EP 3137220 schützt eine Vorrichtung zur hydrodynamischen Schwerstoffabtrennung bestehend aus Hydrozyklon, Klassierrohr und Speicherkammer mit einem gesteuerten Spülwasserstrom. Der Verfahrensanspruch bezieht sich auf die Regelung des Spülwasserstroms, der Detektion des Füllstands der Speicherkammer und das gesteuerte Fluten der Speicherkammer.

Eine wichtige Einflussgröße auf die Abscheidung im Hydrozyklon ist die dynamische Viskosität der Suspension (Trawinski, H. (1995) Der Trennvorgang im Hydrozyklon. Aufbereitungstechnik 36, 410-417). Abfallsuspensionen sind häufig strukturviskos und es besteht ein Zusammenhang zwischen Viskosität und Trockenrückstand bzw. Feststoffgehalt, wenn die Suspensionen aus dem gleichen

Abfall erzeugt werden. Versuche ergaben für unterschiedliche Trockenrückstände einer Suspension folgenden Zusammenhang zwischen dynamischer Viskosität η und Scherrate γ:

| Trockenrückstand [% Feuchtmasse] | dynamische Viskosität η [mPas] |
|---|---|
| 11,4 | 21900 * γ [s⁻¹]^{-0,884} |
| 13,5 | 34700 * γ [s⁻¹]^{-0,765} |
| 18,8 | 21900 * γ [s⁻¹]^{-0,706} |

Für einen Hydrozyklon wurde mit einer Abfallsuspension der Einfluss des Feststoffgehalts der Suspension und des Volumenstroms auf die Trenneffizienz untersucht (Do Carmo Precci Lopes, A., Senfter, T., Ebner, C., Senn, M., Pillei, M., Kraxner, M., Robra, S. Bockreis, A. (2021) Separation of biodegradable material from low calorific fraction of municipal solid waste. Journal of Cleaner Production 280, 124681). Diese Untersuchungen zeigen, dass Feststoffgehalt (Trockenrückstand) der Suspension, Volumenstrom und Anzahl der Durchläufe durch den Hydrozyklon wesentlich den Abtrenngrad der Schwerstoffe beeinflussen.

Bei einer hydrodynamischen Schwerstoffabtrennung gemäß EP 3137220 wurde in einer Abfallvergärungsanlage mit einer nassmechanischen Aufbereitung für einen Abfalldurchsatz von 12 Mg/h die Reduktion der anorganischen sedimentierenden Bestandteile einer Abfallsuspension mit einem Trockenrückstand von 10,5 bis 11,5 % bezogen auf die Feuchtmasse bestimmt. Die Ergebnisse bestätigen, dass die Anzahl der Durchläufe den Abtrenngrad beeinflusst.

| | Anzahl Durchläufe | Anorganische sedimentierende Bestandteile [g/kg] | | | |
|---|---|---|---|---|---|
| | | > 2 mm | > 1 mm | > 450 µm | > 150 µm |
| Zulauf | | 0,942 | 1,684 | 2,246 | 2,830 |
| Ablauf | 4,7 | 0,119 | 0,265 | 0,442 | 0,749 |
| Zulauf | | 0,803 | 1,575 | 2,251 | 2,903 |
| Ablauf | 5,6 | 0,055 | 0,175 | 0,387 | 0,687 |

Bei der Aufbereitung von feuchten Abfallfraktionen mittels Separationszerkleinerer oder Pressen für eine anschließende Vergärung der vergärbaren Bestandteile werden Abfallsuspensionen mit einem Trockenrückstand von bis zu 20 % bezogen auf deren Feuchtmasse erzeugt. Suspensionen mit derartig hohem Trockenrückstand bzw. Feststoffgehalt weisen für eine effiziente Abtrennung der Schwerstoffe mittels Hydrozyklon eine zu hohe Viskosität auf. Eine Reduktion der Viskosität einer Abfallsuspension durch Verdünnen ermöglicht einen effizienten Einsatz einer hydrodynamischen Schwerstoffabtrennung. Ein Verdünnen mit zusätzlichem Wasser vergrößert jedoch das Volumen der Abfallsuspension und verursacht somit eine Vergrößerung des erforderlichen Volumens des nachgeschalteten Vergärungsreaktors.

Bei der Vergärung der Abfallsuspension werden vergärbare Bestandteile in Biogas umgewandelt, das abgeführt wird. Somit weist der Inhalt eines Vergärungsreaktors im Vergleich zur zugeführten Abfallsuspension einen deutlich geringeren Trockenrückstand bzw. geringere Viskosität auf. Vergärungsreaktoren werden in der Regel mit Umwälzung ausgeführt, der die Abfallsuspension zugeführt wird. Durch dieses Mischen der Abfallsuspension mit der Reaktorumwälzung kann die Viskosität beeinflusst werden. Wird die hydrodynamische Schwerstoffabtrennung in die Umwälzung des Vergärungsreaktors integriert, kann durch das Mischungsverhältnis von Abfallsuspension zu Reaktorumwälzung die Viskosität im Zulauf zum Hydrozyklon gesteuert werden. Dieser Ansatz wurde bei einer Anlage zur Co-Vergärung einer Abfallsuspension mit Klärschlamm umgesetzt (Eisendle, R., Niederkircher, A., Ebner, C. (2016) Neuartige Zyklontechnik für die Abtrennung von Störstoffen. KA Betriebs-Info 46, 2444-2446). Diese Ausführung hat jedoch den Nachteil, dass vor dem Eintritt in den Vergärungsreaktor nur ein Durchgang der Abfallsuspension durch den Hydrozyklon möglich ist. Dies führt dazu, dass nur ein Teil der sedimentierenden Schwerstoffe abgetrennt wird und sich bei der deutlich geringeren Viskosität des Inhalts des Vergärungsreaktors verstärkt Sedimente im Behälter bilden.

Um die Abtrennung im Hydrozyklon zu optimieren, wird die Zufuhr zum Hydrozyklon über den Druckverlust im Hydrozyklon gesteuert. Dies legt den Volumenstrom zum Schwerstoffabscheider fest. Um einen Zielwert für die Viskosität bzw. den Feststoffgehalt in der Zufuhr realisieren zu können, muss der Volumenstrom der zugeführten Abfallsuspension an die Zufuhr zum Hydrozyklon angepasst werden. Um dadurch eine Rückwirkung auf die Abfallaufbereitung zu verhindern, muss ein Speicherbehälter zwischengeschaltet werden. Abfallsuspensionen mit Trockenrückständen von bis zu 20 % der Feuchtmasse verhalten sich jedoch zäh fließend. Deshalb ist deren Speicherung in einem Behälter nur aufwendig zu realisieren und bleibt trotzdem problematisch.

Im Gegensatz dazu gewährleistet das erfindungsgemäße Verfahren einen optimalen Betrieb eines Hydrozyklons als Bestandteil einer Schwerstoffabscheidung. Ohne einschränkende Rückwirkung auf die vorgeschaltete Verfahrensstufe zur Erzeugung der Aufschlämmung kann der Feststoffgehalt in der Zufuhr zum Hydrozyklon durch gesteuerte Rückführung aus einem Vergärungsreaktor als auch die Anzahl der Durchläufe eingestellt werden.

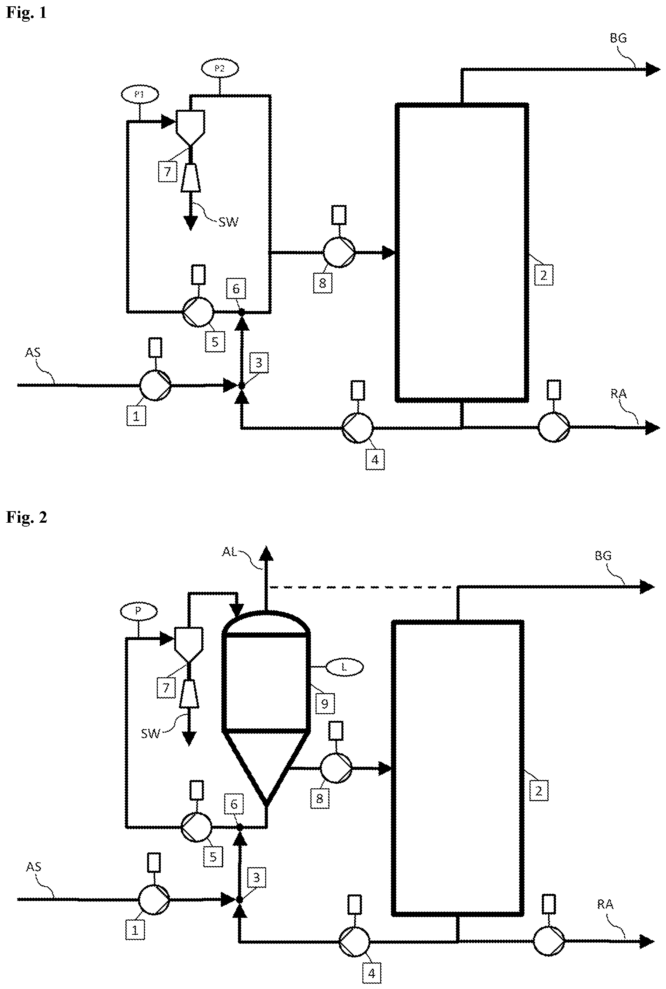

In Fig. 1 ist eine entsprechende Verfahrensführung dargestellt. Die Aufschlämmung (Abfallsuspension) wird mittels der Zufuhrpumpe (1), deren Durchsatz entsprechend dem Anfall an Abfallsuspension geregelt wird, in eine Rückführung aus dem Vergärungsreaktor (2) gepumpt (3). Der Durchsatz der Rückführpumpe (4) wird auf Basis eines vorgegebenen Verhältnisses zum Durchsatz der Zufuhrpumpe (1) gesteuert, um die erforderliche Reduktion des Feststoffgehalts zu erreichen. Das dadurch erzeugte Gemisch, das zum Vergleich mit der Abfallsuspension einen niedrigeren Feststoffgehalt hat, wird der Saugseite der Beschickungspumpe des Hydrozyklons (5) zugeführt (6). Die Durchsätze des Hydrozyklons (7) sowie dessen Beschickungspumpe sind ein Mehrfaches der Summe der Durchsätze der Zufuhrpumpe (1) und der Rückführpumpe (4) zusammen, um mehrere Durchläufe der Abfallsuspension durch die Schwerstoffabscheidung zu ermöglichen, bevor diese dem Vergärungsreaktor zugeführt wird. Der Durchsatz der Beschickungspumpe (5) wird mittels Druckmessung (P1) im Zulauf des Hydrozyklons (7) geregelt. Die im Hydrozyklon von Schwerstoffen entfrachtete Suspension wird auf die Saugseite der Beschickungspumpe des Hydrozyklons (5) zurückgeführt. Um im Hydrozyklon einen konstanten Druckverlust zu gewährleisten, wird die Förderleistung der Beschickungspumpe des Vergärungsreaktors (8) mittels der Druckmessung (P2) im Ablauf des Hydrozyklons (7) so geregelt, dass die Druckdifferenz P1-P2 konstant ist.

Die Einbindung eines Speichers in den Ablauf des Hydrozyklons ermöglicht eine stabilere Regelung (siehe Fig. 2). Durch den freien Überlauf des Hydrozyklons (7) in den Speicher (9) ist zur Gewährleistung eines konstanten Druckverlusts im Hydrozyklon nur eine Druckmessung (P) im Zulauf des Hydrozyklons erforderlich. Sofern der Füllstand (L) im Speicher (9) es zulässt, wird die Beschickungspumpe des Vergärungsreaktors (8) auf Basis eines vorgegebenen Verhältnisses zum Durchsatz der Beschickungspumpe des Hydrozyklons (5) gesteuert, um eine vorgegebene Anzahl von Durchläufen durch die Schwerstoffabscheidung zu realisieren. Wird im Speicher (9) ein vorgegebener oberer Füllstand überschritten, wird der Durchsatz der Beschickungspumpe des Vergärungsreaktors (8) in Abhängigkeit des Füllstands (L) im Speicher (9) geregelt bis der obere Füllstand im Speicher (9) wieder unterschritten wird. Dann wird der Durchsatz der Beschickungspumpe des Vergärungsreaktors (8) wieder auf Basis des Durchsatzes der Beschickungspumpe der Schwerstoffabscheidung (5) gesteuert.

Der Speicher (9) ist präferiert an die Abluftbehandlung angeschlossen. Ist aufgrund einer anaeroben biologischen Aktivität im Speicher mit der Bildung relevanter Mengen an Methan oder Wasserstoff zu rechnen, ist der Speicher an die Biogaserfassung anzuschließen (Fig. 2 gestrichelte Linie), um den Biogasertrag aus der Vergärung zu verbessern.

Die Durchsätze der Pumpen werden über die Drehzahl der jeweiligen Pumpe gesteuert. In der einfachen Ausführung des Verfahrens mit Speicher werden die Drehzahlvorgaben wie folgt bestimmt:
- Die Drehzahl der Zufuhrpumpe (1) wird in Abhängigkeit des anfallenden Volumens an Abfallsuspension gesteuert.
- Die erforderliche Drehzahl der Rückführpumpe (4) wird bestimmt auf Grundlage von:
   - Durchsatz der Zufuhrpumpe berechnet mittels Drehzahl der Zufuhrpumpe, aus Bestand ermittelter Förderhöhe und der in der Steuerung hinterlegten Pumpenkennlinie,
   - erforderlicher Durchsatz der Rückführpumpe, der mittels Verhältnis der mittleren Trockenrückstände in der Abfallsuspension und der Rückführung (Trockenrückstände in Proben im Labor bestimmt) sowie dem Trockenrückstand, der in der Mischung dieser beiden Stoffströme erreicht werden soll, berechnet wird und
   - erforderliche Drehzahl der Rückführpumpe, die aus dem erforderlichen Durchsatz mittels festgelegter Förderhöhe und der in der Steuerung hinterlegten Pumpenkennlinie berechnet wird.
- Die Drehzahl der Beschickungspumpe der Schwerstoffabscheidung (5) wird mittels der Druckmessung im Zulauf des Hydrozyklons (P) geregelt, um den vorgegebenen Druckverlust im Hydrozyklon zu gewährleisten.
- Die erforderliche Drehzahl der Beschickungspumpe des Vergärungsreaktors (8) wird im Fall, dass der Füllstand im Speicher (9) unterhalb eines vorgegebenen oberen Füllstands liegt, bestimmt auf Grundlage von:
   - Durchsatz der Beschickungspumpe des Hydrozyklons (5) wird berechnet mittels Drehzahl der Beschickungspumpe, aus Bestand ermittelter Förderhöhe, Druckverlust im Hydrozyklon und der in der Steuerung hinterlegten Pumpenkennlinie,
   - erforderlicher Durchsatz der Beschickungspumpe des Vergärungsreaktors (8), der mittels dem Durchsatz der Beschickungspumpe des Hydrozyklons (5) und der vorgegebenen Anzahl an Durchläufen durch den Hydrozyklon berechnet wird und
   - erforderliche Drehzahl der Beschickungspumpe des Vergärungsreaktors (8), die aus dem erforderlichen Durchsatz mittels aus Bestand ermittelter Förderhöhe und der in der Steuerung hinterlegten Pumpenkennlinie berechnet wird.
- Die erforderliche Drehzahl der Beschickungspumpe des Vergärungsreaktors (8) wird im Fall, dass der Füllstand im Speicher (9) oberhalb eines vorgegebenen oberen Füllstands liegt, mittels der Füllstandmessung (L) im Speicher derart geregelt, dass der Füllstand langsam absinkt, bis dieser unter dem vorgegebenen oberen Füllstand liegt.

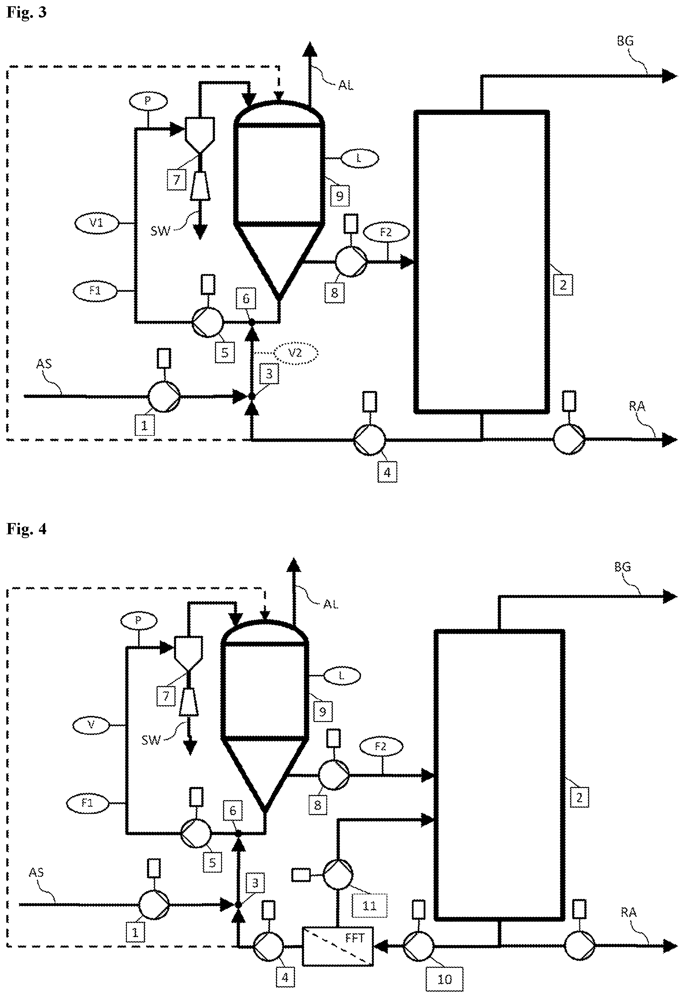

Eine bevorzugte Ausführung des Verfahrens ist mit zusätzlicher Messtechnik ausgestattet (Fig. 3). In der Zufuhr zum Hydrozyklon wird der Feststoffgehalt der Suspension (V1) gemessen als Orientierung für den Trockenrückstand bzw. die Viskosität der Suspension. In Abhängigkeit von diesem Messwert wird die Drehzahl der Rückführpumpe (4) so geregelt, dass der gemessene Feststoffgehalt dem der Steuerung vorgegebenen Sollwert entspricht. Alternativ kann der Feststoffgehalt auch in der Mischung von Abfallsuspension und Rückführung (V2) gemessen werden, wenn dies ein besseres Regelverhalten ermöglicht. Ferner sind Durchflussmessungen in der Zufuhr zum Hydrozyklon (F1) und in der Zufuhr zum Vergärungsreaktor (F2) installiert. Dadurch kann im Betriebsfall, dass der Füllstand im Speicher (9) unterhalb eines vorgegebenen oberen Füllstands liegt, die Drehzahl der Beschickungspumpe des Vergärungsreaktors (8) so geregelt werden, dass ein vorgegebenes Verhältnis von F1 zu F2 eingehalten wird. Dieses Verhältnis entspricht ungefähr der Anzahl der Durchläufe durch die Schwerstoffabscheidung, da die Masse der abgeschiedenen Schwerstoffe (SW) im Vergleich zu den Massen der Volumenströmen F1 und F2 vernachlässigbar ist.

Zum Einstellen des Trockenrückstands, der für eine optimale Schwerstoffabscheidung erforderlich ist, kann aufgrund des Trockenrückstands im Vergärungsreaktor die erforderliche Rückführung sehr hoch werden. Wird dadurch die hydraulische Belastung des Hydrozyklons zu hoch, ist ein zweiter Schwerstoffabscheider erforderlich. In diesem Fall kann der Trockenrückstand im zurückzuführenden Volumenstrom mit einer Fest-Flüssig-Trennung reduziert werden. Fig. 4 zeigt das erfindungsgemäße Verfahren mit den erforderlichen Ergänzungen. Aus dem Vergärungsreaktor (2) wird mit einer Pumpe (10) eine Fest-Flüssig-Trennung (FFT) beschickt, die einen großen Teil der Feststoffe, die hauptsächlich zu einer höheren Viskosität beitragen, in einer noch pumpbaren Konsistenz abtrennt. Diese werden dann mit einer Pumpe (11) in den Vergärungsreaktor zurückgeführt. Mit der Rückführpumpe (4) wird der erzeugte Stoffstrom, der sich im Vergleich zum Inhalt des Vergärungsreaktors durch einen geringeren Trockenrückstand bzw. eine geringere Viskosität auszeichnet, zum Verschneiden mit der Abfallsuspension (3) zurückgeführt. Aufgrund des reduzierten Trockenrückstands des zur Schwerstoffabscheidung zurückgeführten Materials aus dem Vergärungsreaktor muss zum Einstellen des erforderlichen Trockenrückstands im Zulauf des Hydrozyklons (7) die Rückführpumpe (4) weniger der Schwerstoffabscheidung zuführen. Dadurch kann durch gleichbleibende hydraulische Belastung des Hydrozyklons eine Vergrößerung der Schwerstoffabscheidung vermieden oder die vorgegebene Anzahl der Durchgänge realisiert werden.

Durch die Rückführung einer durch die Fest-Flüssig-Trennung von abfiltrierbaren Stoffen entfrachteten Flüssigphase mit der Rückführpumpe (4) wird auch der Eintrag von methanogenen Mikroorganismen in den Speicher (9) reduziert. Dadurch wird die Bildung von Methan und Wasserstoff im Speicher weitgehend unterbunden. Dies begünstigt einen Anschluss des Speichers an die Abluftbehandlung, der betriebstechnisch Vorteile bringt und kostengünstiger zu realisieren ist.

Ein Vorteil der oben beschriebenen Verfahrensführungen ist, dass die Verdünnung der Abfallsuspension mit Inhalt des Vergärungsreaktors erfolgt. Somit hat diese weder einen Einfluss auf die hydraulische Verweilzeit noch auf die Feststoffverweilzeit im Vergärungsreaktor. Der Vergärungsreaktor kann somit ausschließlich auf den deutlich kleineren Anfall an Abfallsuspension ausgelegt werden.

Das erfindungsgemäße Verfahren kann wie folgt realisiert werden:
Bei der zuvor erwähnten Abfallvergärungsanlage liegt der Trockenrückstand der Abfallsuspension bei ca. 11 % bezogen auf die Feuchtmasse (FM) bei einem Anfall von 37 Mg/h. Der Trockenrückstand im Vergärungsreaktor beträgt ca. 6 %FM. Do Carmo Precci Lopes et al. (2021) empfehlen in ihrer Veröffentlichung im Zulauf des Hydrozyklons einen Trockenrückstand von 9 %FM. Dieser Trockenrückstand kann bei der zu Fig. 1 beschriebenen Verfahrensführung nach dem Zusammenführen von Abfallsuspension und Rückführung in Punkt 3 durch Rückführen von 25 Mg Inhalt des Vergärungsreaktors pro Stunde eingestellt werden. Sofern durch die Schwerstoffabscheidung der Trockenrückstand im Zu- und Auslauf des Hydrozyklons sich nur vernachlässigbar ändert, entspricht der Trockenrückstand in der Zuführung zum Hydrozyklon diesem Wert. Bei einem Durchsatz von 310 Mg/h durch die Schwerstoffabscheidung können 5 Durchläufe durch den Hydrozyklon realisiert werden.

Ändert sich der Trockenrückstand beim Durchgang durch den Hydrozyklon wesentlich, ist eine Verfahrensführung analog zu Fig. 3 vorzuziehen. Trennt die Schwerstoffabtrennung 10 % des Trockenrückstandes der Abfallsuspension ab, muss die Rückführpumpe (4) nur 15 Mg Inhalt des Vergärungsreaktors pro Stunde fördern, um im Zulauf des Hydrozyklons den angestrebten Trockenrückstand vom 9 %FM einzuhalten. In diesem Fall müssen für 5 Durchläufe dem Hydrozyklon nur 260 Mg/h zugeführt werden.

Werden dieser Verfahrensführung 37 Mg/h einer Abfallsuspension mit einem Trockenrückstand von 17 %FM zugeführt, und davon 10 % in der Schwerstoffabscheidung abgetrennt, müssen bei einem Trockenrückstand von 6 %FM im Vergärungsreaktor 88 Mg/h zurückgeführt werden, um im Zulauf des Hydrozyklons einen Trockenrückstand von 9 %FM einzustellen. Um eine Anzahl von fünf Durchläufen durch den Hydrozyklon zu erreichen, müssen diesem 625 Mg/h zugeführt werden.

Erfolgt in diesem Fall die Verfahrensführung analog zu Fig. 4, und kann mit der FFT der Trockenrückstand in der Rückführung auf 2 %FM abgesenkt werden, müssen zum Einstellen des Trockenrückstands im Zulauf Hydrozyklon auf 9 %FM nur 36 Mg/h zurückgeführt werden. Dadurch ermöglicht bereits ein Durchsatz von 365 Mg/h durch die Schwerstoffabscheidung eine Anzahl von fünf Durchläufen.

Da die Anzahl der erforderlichen Durchläufe durch den Hydrozyklon maßgeblich von dem Verhältnis des Zufuhrstroms zur Schwerstoffabscheidung zum Beschickungsstrom zur Vergärung bestimmt wird, hat das Nutzvolumen des Speichers (9) darauf keinen Einfluss. Aufgrund des Mischens der Abfallsuspension mit Inhalt des Vergärungsreaktors zeichnet sich diese Mischung durch eine methanogene Aktivität aus. Um die Biogasbildung im Speicher zu begrenzen, wird das Nutzvolumen des Speichers begrenzt, um eine möglichst kurze Verweilzeit zu gewährleisten. Bei einer präferierten Ausführung des erfindungsgemäßen Verfahrens beträgt das Nutzvolumen des Speichers nur das 1- bis 2-fache des pro Stunde zugeführten Volumens an Abfallsuspension.

Als Zufuhrpumpe (1) und Beschickungspumpe der Schwerstoffabtrennung (5) werden bevorzugt Zentrifugalpumpen eingesetzt, da diese in sehr robuster und verschleißarmer Ausführung verfügbar sind. Aufgrund des hydrostatischen Drucks im Vergärungsreaktor und des reduzierten Gehalts an Schwerstoffen in den zu fördernden Stoffströmen werden die Beschickungspumpe Vergärungsreaktor (8) und die Rückführpumpe (4), sofern diese direkt aus dem Reaktor abzieht, als Verdrängerpumpen ausgeführt. Die Ausführung der Rückführpumpe als Verdrängerpumpe kann bei einer Verfahrenskonzeption nach Fig. 1 oder Fig. 2 je nach Betriebs- und Einbausituation erfordern, dass die Zufuhrpumpe als Verdrängerpumpe ausgeführt werden muss. Bei der Verfahrenskonzeption nach Fig. 3 kann dies vermieden werden. Wird die Rückführpumpe mittels der Messung des Feststoffgehalts der Suspension (V1) im Zulauf Hydrozyklon gesteuert, kann die Rückführung direkt (gestrichelte Linie) dem Speicher (9) zugeführt werden.

Bei einer Verfahrensführung analog Fig. 4 sind FFT-Beschickungspumpe (10) und Pumpe zur Rückführung der Feststoffe (11) Verdrängerpumpen. In diesem Fall kann die Rückführpumpe (4) als kostengünstigere und verschleißarme Zentrifugalpumpe ausgeführt werden. Eine Rückführung (gestrichelte Linie) direkt in den Speicher (9) vereinfacht die Auslegung der Rückführpumpe und verbessert das Regelverhalten, da der Gegendruck der Rückführpumpe sich nur in Abhängigkeit der Förderleistung dieser Pumpe ändert.

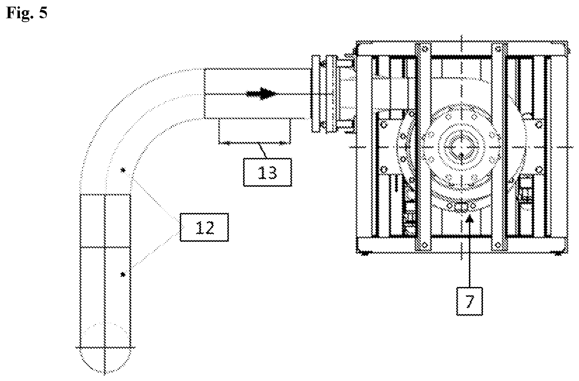

In der Zuführung zum Hydrozyklon bewirken zwei 90°-Bögen (12), dass der Zufuhrstrom vor Eintritt in den Hydrozyklon von der vertikalen Fließrichtung in eine horizontale Fließrichtung und anschließend horizontal umgelenkt wird. Dies stellt sicher, dass die Schwerstoffe zum Großteil tangential an der Wand des Hydrozyklons eingeleitet werden. Der Sensor der Druckmessung wird in die Zufuhrleitung seitlich kurz vor dem Hydrozyklon auf der gegenüberliegenden Seite (13) eingebaut, um die Abrasion zu reduzieren. Ferner ragt dieser nicht in die Suspensionsströmung, sondern wird wenige Millimeter von der Rohrinnenwand zurück versetzt eingebaut, um seine Standzeiten zu verbessern (Fig. 5).

Die Zufuhr des Gemisches von Aufschlämmung und Rückführung aus dem Vergärungsreaktor erfolgt auf der Saugseite Beschickungspumpe der Schwerstoffabtrennung (5). Dies stellt sicher, dass durch diese Zentrifugalpumpe eine gute Durchmischung der drei Stoffströme erfolgt, die dem Hydrozyklon zugeführt werden.

### Bezugszeichenliste:

- Fig. 1:: Schema einer einfachen Verfahrensführung
- Fig. 2:: Schema einer einfachen Verfahrensführung mit Speicher
- Fig. 3:: Schema der präferierten Verfahrensführung
- Fig. 4:: Verfahrensführung mit Fest-Flüssig-Trennung
- Fig. 5:: Zuführung Hydrozyklon
- AL: Abluft
- AS: Aufschlämmung / Abfallsuspension
- BG: Biogas
- FFT: Fest-Flüssig-Trennung
- RA: Reaktoraustrag
- SW: Schwerstoffe
- F: Durchflussmessung
- L: Füllstandmessung
- P: Druckmessung
- V: Feststoffgehaltmessung
- 1: Zufuhrpumpe
- 2: Vergärungsreaktor
- 3: Einmischung Aufschlämmung
- 4: Rückführpumpe
- 5: Beschickungspumpe Hydrozyklon
- 6: Einmischung Saugseite Beschickungspumpe
- 7: Hydrozyklon
- 8: Beschickungspumpe Vergärungsreaktor
- 9: Speicher
- 10: Beschickungspumpe Fest-Flüssig-Trennung
- 11: Schlammrückführpumpe Vergärungsreaktor
- 12: 90°-Bögen
- 13: Installationsbereich Drucksensor

## Patentansprüche

1. Verfahren zur hydrodynamischen Abtrennung von Schwerstoffen einer Aufschlämmung (AS) mit Bestandteilen unterschiedlicher Dichte mittels Hydrozyklons (7), welche Aufschlämmung nachfolgend der Schwerstoffabtrennung für eine Vergärung vergärbarer Bestandteile dieser Aufschlämmung einem Vergärungsreaktor (2) zugeführt wird, wobei die Aufschlämmung (AS) zur Bildung einer Substrat-Zufuhr zum Hydrozyklon mit dem Material einer gesteuerten Rückführung von Inhalt aus dem Vergärungsreaktor (2) gemischt wird, um eine Reduktion an Feststoffgehalt zu erreichen, **dadurch gekennzeichnet, dass** der Substrat-Zufuhr zur Beschickung des Hydrozyklons (7) weiters eine von Schwerstoffen entfrachtete Suspension aus dem Hydrozyklon mittels Rückführung vom Hydrozyklon in das Gemisch aus Aufschlämmung und Vergärungsreaktor-Inhalt zugemischt wird, um mehrere Durchläufe der Aufschlämmung durch den Hydrozyklon zu ermöglichen, bevor diese dem Vergärungsreaktor zugeführt wird, derart, dass der Zufuhrstrom zur Schwerstoffabtrennung mindestens um das Zweifache größer ist als der Massestrom des Gemisches aus Aufschlämmung und Rückführung aus dem Vergärungsreaktor und der Durchsatz durch den Hydrozyklon mittels Messung des Drucks (P1) im Zulauf des Hydrozyklons geregelt wird, derart, dass eine Druckdifferenz P1-P2 konstant gehalten wird, wobei die Förderleistung der Beschickungspumpe des Vergärungsreaktors (8) mittels der Druckmessung (P2) im Ablauf des Hydrozyklons (7) so geregelt wird, dass die Druckdifferenz P1-P2 konstant ist.

2. Verfahren nach Anspruch 1, **dadurch gekennzeichnet, dass** die von Schwerstoffen entfrachtete Suspension aus dem Hydrozyklon vor der Zufuhr in den Vergärungsreaktor oder vor der Zufuhr in das Gemisch aus Aufschlämmung und Rückführung aus dem Vergärungsreaktor in einem Speicher (9) gespeichert wird, wobei zur Gewährleistung eines konstanten Druckverlusts im Hydrozyklon eine Druckmessung (P) im Zulauf des Hydrozyklons erfolgt.

3. Verfahren nach Anspruch 1 oder 2, **dadurch gekennzeichnet, dass** der Rückführstrom aus dem Vergärungsreaktor (2) in Abhängigkeit der Drehzahl der Zufuhrpumpe der Aufschlämmung, des Feststoffgehalts der Aufschlämmung und des Feststoffgehalts des Rückführstroms aus dem Vergärungsreaktor (2) gesteuert wird.

4. Verfahren nach Anspruch 1 oder 2 **dadurch gekennzeichnet, dass** der Rückführstrom aus dem Vergärungsreaktor (2) mittels einer Messung des Feststoffgehalts in der Zuführung zur Schwerstoffabtrennung gesteuert wird.

5. Verfahren nach Anspruch 1 bis 4, **dadurch gekennzeichnet, dass** die Zufuhr zum Vergärungsreaktor mittels einer Messung des Zufuhrstroms zur Schwerstoffabscheidung gesteuert wird.

6. Verfahren nach Anspruch 1 bis 5, **dadurch gekennzeichnet, dass** der Feststoffgehalt des aus dem Vergärungsreaktor zur Schwerstoffabtrennung zurückgeführten Stoffstroms mittels Fest-Flüssig-Trennung reduziert wird, und die abgetrennten Feststoffe in den Vergärungsreaktor zurückgeführt werden.

7. Vorrichtung zum Verarbeiten einer Aufschlämmung mit Bestandteilen unterschiedlicher Dichte in Verbindung mit einer anaeroben Vergärung vergärbarer Bestandteile der Aufschlämmung in einem Vergärungsreaktor (9) und einem zum hydrodynamischen Abtrennen von Schwerstoffen der Aufschlämmung vorgesehenen Hydrozyklon, welche Vorrichtung eine Substrat-Zufuhr-Leitung zum Hydrozyklon aufweist, in die
a) eine Gemisch-Leitung aus einerseits Aufschlämmung und andererseits Rückführ-Inhalt aus dem Vergärungsreaktor (2) mündet, und
b) eine Suspensions-Leitung für eine von Schwerstoffen entfrachtete Suspension aus dem Hydrozyklon,
womit mehrere Durchläufe der Aufschlämmung durch den Hydrozyklon erwirkbar sind, **dadurch gekennzeichnet, dass** in der Substrat-Zufuhr-Leitung zum Hydrozyklon der Durchsatz durch den Hydrozyklon mittels Messung des Drucks (P1) im Zulauf des Hydrozyklons geregelt wird, derart, dass eine Druckdifferenz P1-P2 konstant gehalten wird, wobei die Förderleistung der Beschickungspumpe des Vergärungsreaktors (8) mittels der Druckmessung (P2) im Ablauf des Hydrozyklons (7) so geregelt wird, dass die Druckdifferenz P1-P2 konstant ist.

8. Vorrichtung nach Anspruch 7, **dadurch gekennzeichnet, dass** in der Zuführung zum Schwerstoffabscheider ein Sensor zum Messen des Feststoffgehalts installiert ist, der die Förderleistung der Rückführpumpe aus dem Vergärungsreaktor steuert.

9. Vorrichtung nach Anspruch 7 oder 8, **dadurch gekennzeichnet, dass** in der Zuführung zum Schwerstoffabscheider und/oder in der Zuführung zum Vergärungsreaktor (ein) Durchflussmesser installiert ist/sind.

10. Vorrichtung nach einem der Ansprüche 7 bis 9, **dadurch gekennzeichnet, dass** die Aufschlämmung vor Eintritt in den Hydrozyklon (7) mit einem 90°-Bogen von der vertikalen Fließrichtung in eine horizontale Fließrichtung und anschließend mit einem 90°-Bogen horizontal umgelenkt wird, und der Drucksensor zum Steuern der Beschickungspumpe auf der Innenseite des durch den zweiten Bogen erzeugten Strömungsprofils installiert ist.

11. Vorrichtung nach einem der Ansprüche 7 bis 10, **dadurch gekennzeichnet, dass** ein Speicher (9) in den Ablauf des Hydrozyklons eingebunden ist.

12. Vorrichtung nach Anspruch 11, **dadurch gekennzeichnet, dass** der Speicher (9) an die Abluftbehandlung angeschlossen ist.

13. Vorrichtung nach einem der Ansprüche 7 bis 12, **dadurch gekennzeichnet, dass** der Durchsatz durch den Hydrozyklon mittels Messung des Drucks (P1) im Zulauf des Hydrozyklons und des Drucks (P2) im Ablauf des Hydrozyklons geregelt wird, derart, dass eine Druckdifferenz (P1-P2) konstant bleibt.

14. Vorrichtung nach einem der Ansprüche 7 bis 13, **dadurch gekennzeichnet, dass** zur Gewährleistung eines konstanten Druckverlusts im Hydrozyklon eine Druckmessung (P) im Zulauf des Hydrozyklons erfolgt.

## Claims

1. Process for the hydrodynamic separation of heavy materials from a slurry (AS) comprising components of different densities by means of a hydrocyclone (7), which slurry is subsequently fed to a digestion reactor (2) for anaerobic digestion of digestible components of said slurry following the heavy material separation, wherein the slurry (AS) is mixed with the material of a controlled recirculation of content from the digestion reactor (2) to form a substrate feed to the hydrocyclone in order to achieve a reduction in solids content, **characterised in that** a heavy-material-depleted suspension from the hydrocyclone is additionally admixed into the substrate feed for charging the hydrocyclone (7) by means of recirculation from the hydrocyclone into the mixture of slurry and digestion reactor content, in order to enable multiple passes of the slurry through the hydrocyclone before it is fed to the digestion reactor, in such a manner that the feed flow for the heavy material separation is at least twice as large as the mass flow rate of the mixture of slurry and recirculation from the digestion reactor, and the throughput through the hydrocyclone is controlled by means of measurement of the pressure (P1) at the inlet of the hydrocyclone, in such a manner that a pressure difference P1-P2 is kept constant, wherein the pumping capacity of the feed pump of the digestion reactor (8) is controlled by means of the pressure measurement (P2) at the outlet of the hydrocyclone (7) such that the pressure difference P1-P2 is constant.

2. Process according to claim 1, **characterised in that** the heavy-material-depleted suspension from the hydrocyclone is stored in a storage tank (9) prior to being fed to the digestion reactor or prior to being fed to the mixture of slurry and recirculation from the digestion reactor, wherein, in order to ensure a constant pressure drop in the hydrocyclone, a pressure measurement (P) is carried out at the inlet of the hydrocyclone.

3. Process according to claim 1 or 2, **characterised in that** the recirculation flow from the digestion reactor (2) is controlled in dependence on the rotational speed of the feed pump of the slurry, the solids content of the slurry, and the solids content of the recirculation flow from the digestion reactor (2).

4. Process according to claim 1 or 2, **characterised in that** the recirculation flow from the digestion reactor (2) is controlled by means of a measurement of the solids content in the feed to the heavy material separation.

5. Process according to claims 1 to 4, **characterised in that** the feed to the digestion reactor is controlled by means of a measurement of the feed flow to the heavy material separation.

6. Process according to claims 1 to 5, **characterised in that** the solids content of the flow recirculated from the digestion reactor for the heavy material separation is reduced by means of solid-liquid separation, and the separated solids are returned to the digestion reactor.

7. Device for processing a slurry comprising components of different densities in connection with anaerobic digestion of digestible components of the slurry in a digestion reactor (9) and a hydrocyclone provided for hydrodynamic separation of heavy materials from the slurry, which device has a substrate feed line to the hydrocyclone, into which
a) a mixing line opens from slurry on the one hand and recirculation content from the digestion reactor (2) on the other hand, and
b) a suspension line for a heavy-material-depleted suspension from the hydrocyclone,
whereby multiple passes of the slurry through the hydrocyclone can be effected, **characterised in that** in the substrate feed line to the hydrocyclone the throughput through the hydrocyclone is controlled by means of measurement of the pressure (P1) at the inlet of the hydrocyclone, in such a manner that a pressure difference P1-P2 is kept constant, wherein the pumping capacity of the feed pump of the digestion reactor (8) is controlled by means of the pressure measurement (P2) at the outlet of the hydrocyclone (7) such that the pressure difference P1-P2 is constant.

8. Device according to claim 7, **characterised in that** a sensor for measuring the solids content is installed in the feed to the heavy material separator, which controls the pumping capacity of the recirculation pump from the digestion reactor.

9. Device according to claim 7 or 8, **characterised in that** a flow meter or flow meters is/are installed in the feed to the heavy material separator and/or in the feed to the digestion reactor.

10. Device according to one of claims 7 to 9, **characterised in that** the slurry is deflected by a 90° bend from the vertical flow direction into a horizontal flow direction and subsequently by a 90° bend horizontally prior to entry into the hydrocyclone (7), and the pressure sensor for controlling the feed pump is installed on the inner side of the flow profile generated by the second bend.

11. Device according to one of claims 7 to 10, **characterised in that** a storage tank (9) is incorporated into the outlet of the hydrocyclone.

12. Device according to claim 11, **characterised in that** the storage tank (9) is connected to the exhaust air treatment system.

13. Device according to one of claims 7 to 12, **characterised in that** the throughput through the hydrocyclone is controlled by means of measurement of the pressure (P1) at the inlet of the hydrocyclone and the pressure (P2) at the outlet of the hydrocyclone, in such a manner that a pressure difference (P1-P2) remains constant.

14. Device according to one of claims 7 to 13, **characterised in that**, in order to ensure a constant pressure drop in the hydrocyclone, a pressure measurement (P) is carried out at the inlet of the hydrocyclone.

## Revendications

1. Procédé de séparation hydrodynamique des matières lourdes d'une bouillie (AS) comprenant des constituants de densités différentes au moyen d'un hydrocyclone (7), laquelle bouillie est ensuite acheminée vers un réacteur de méthanisation (2) pour la méthanisation des constituants fermentescibles de ladite bouillie après la séparation des matières lourdes, la bouillie (AS) étant mélangée avec le matériau d'une recirculation commandée de contenu provenant du réacteur de méthanisation (2) pour former une alimentation en substrat vers l'hydrocyclone afin d'obtenir une réduction de la teneur en matières solides, **caractérisé en ce qu'**une suspension débarrassée des matières lourdes provenant de l'hydrocyclone est en outre ajoutée à l'alimentation en substrat pour l'approvisionnement de l'hydrocyclone (7) au moyen d'une recirculation de l'hydrocyclone vers le mélange de bouillie et de contenu du réacteur de méthanisation, afin de permettre plusieurs passages de la bouillie à travers l'hydrocyclone avant qu'elle ne soit acheminée vers le réacteur de méthanisation, de telle sorte que le flux d'alimentation vers la séparation des matières lourdes est au moins deux fois supérieur au débit massique du mélange de bouillie et de la recirculation provenant du réacteur de méthanisation, et le débit à travers l'hydrocyclone est régulé au moyen de la mesure de la pression (P1) à l'admission de l'hydrocyclone, de telle sorte qu'une différence de pression P1-P2 est maintenue constante, le débit de refoulement de la pompe d'alimentation du réacteur de méthanisation (8) étant régulé au moyen de la mesure de pression (P2) à la sortie de l'hydrocyclone (7) de façon que la différence de pression P1-P2 soit constante.

2. Procédé selon la revendication 1, **caractérisé en ce que** la suspension débarrassée des matières lourdes provenant de l'hydrocyclone est stockée dans un réservoir (9) avant d'être acheminée vers le réacteur de méthanisation ou avant d'être acheminée vers le mélange de bouillie et de recirculation provenant du réacteur de méthanisation, une mesure de pression (P) étant effectuée à l'admission de l'hydrocyclone afin de garantir une perte de charge constante dans l'hydrocyclone.

3. Procédé selon la revendication 1 ou 2, **caractérisé en ce que** le flux de recirculation provenant du réacteur de méthanisation (2) est commandé en fonction de la vitesse de rotation de la pompe d'alimentation de la bouillie, de la teneur en matières solides de la bouillie et de la teneur en matières solides du flux de recirculation provenant du réacteur de méthanisation (2).

4. Procédé selon la revendication 1 ou 2, **caractérisé en ce que** le flux de recirculation provenant du réacteur de méthanisation (2) est commandé au moyen d'une mesure de la teneur en matières solides dans l'alimentation vers la séparation des matières lourdes.

5. Procédé selon les revendications 1 à 4, **caractérisé en ce que** l'alimentation vers le réacteur de méthanisation est commandée au moyen d'une mesure du flux d'alimentation vers la séparation des matières lourdes.

6. Procédé selon les revendications 1 à 5, **caractérisé en ce que** la teneur en matières solides du flux recirculé depuis le réacteur de méthanisation vers la séparation des matières lourdes est réduite par séparation solide-liquide, et les matières solides séparées sont renvoyées vers le réacteur de méthanisation.

7. Dispositif pour le traitement d'une bouillie comprenant des constituants de densités différentes en liaison avec la méthanisation anaérobie des constituants fermentescibles de la bouillie dans un réacteur de méthanisation (9) et un hydrocyclone prévu pour la séparation hydrodynamique des matières lourdes de la bouillie, lequel dispositif présente une conduite d'alimentation en substrat vers l'hydrocyclone, dans laquelle débouchent
a) une conduite de mélange composée d'une part de bouillie et d'autre part de contenu en recirculation provenant du réacteur de méthanisation (2), et
b) une conduite de suspension pour une suspension débarrassée des matières lourdes provenant de l'hydrocyclone,
permettant ainsi d'effectuer plusieurs passages de la bouillie à travers l'hydrocyclone, **caractérisé en ce que** dans la conduite d'alimentation en substrat vers l'hydrocyclone, le débit à travers l'hydrocyclone est régulé au moyen de la mesure de la pression (P1) à l'admission de l'hydrocyclone, de telle sorte qu'une différence de pression P1-P2 est maintenue constante, le débit de refoulement de la pompe d'alimentation du réacteur de méthanisation (8) étant régulé au moyen de la mesure de pression (P2) à la sortie de l'hydrocyclone (7) de façon que la différence de pression P1-P2 soit constante.

8. Dispositif selon la revendication 7, **caractérisé en ce qu'**un capteur pour la mesure de la teneur en matières solides est installé dans l'alimentation vers le séparateur de matières lourdes, lequel capteur commande le débit de refoulement de la pompe de recirculation depuis le réacteur de méthanisation.

9. Dispositif selon la revendication 7 ou 8, **caractérisé en ce qu'**un ou plusieurs débitmètre(s) est/sont installé(s) dans l'alimentation vers le séparateur de matières lourdes et/ou dans l'alimentation vers le réacteur de méthanisation.

10. Dispositif selon l'une des revendications 7 à 9, **caractérisé en ce que** la bouillie est déviée, avant son entrée dans l'hydrocyclone (7), par un coude à 90° de la direction d'écoulement verticale vers une direction d'écoulement horizontale, puis déviée horizontalement par un coude à 90°, et le capteur de pression pour la commande de la pompe d'alimentation est installé du côté intérieur du profil d'écoulement engendré par le second coude.

11. Dispositif selon l'une des revendications 7 à 10, **caractérisé en ce qu'**un réservoir (9) est intégré dans la sortie de l'hydrocyclone.

12. Dispositif selon la revendication 11, **caractérisé en ce que** le réservoir (9) est raccordé au traitement de l'air vicié.

13. Dispositif selon l'une des revendications 7 à 12, **caractérisé en ce que** le débit à travers l'hydrocyclone est régulé au moyen de la mesure de la pression (P1) à l'admission de l'hydrocyclone et de la pression (P2) à la sortie de l'hydrocyclone, de telle sorte qu'une différence de pression (P1-P2) demeure constante.

14. Dispositif selon l'une des revendications 7 à 13, **caractérisé en ce qu'**une mesure de pression (P) est effectuée à l'admission de l'hydrocyclone afin de garantir une perte de charge constante dans l'hydrocyclone.
